# EUROPEAN PATENT SPECIFICATION

(11) **EP 2 424 602 B1**
(45) Date of publication and mention of the grant of the patent: **22.07.2015**
(21) Application number: 10769160.2
(22) Date of filing: 03.05.2010
(51) Int. Cl.: A61M 11/00, A61B 18/00, A61N 1/32, A61N 1/04

(54) **SYSTEM FOR DELIVERING A TREATMENT AGENT INTO THE SKIN OF A PATIENT**
SYSTEM ZUR FREISETZUNG EINES THERAPEUTIKUMS IN DIE HAUT EINES PATIENTEN
SYSTÈME D'ADMINISTRATION D'UN AGENT DE TRAITEMENT DANS LA PEAU D'UN PATIENT

(30) Priority: 01.05.2009 AU 2009901909
(43) Date of publication of application: 07.03.2012
(73) Proprietor: Botanical Essentials PTY LTD, Lane Cove West, NSW 2066 (AU)
(72) Inventor: LEVITT, Roger A., Terry Hills, NSW (AU)
(74) Representative: Lau, Sarah Jane
(86) International application number: PCT/AU2010/000509
(87) International publication number: WO 2010/124346

(56) References cited:
- WO-A1-02/18058
- WO-A1-2004/036986
- DE-A1-102006 019 794
- US-A- 5 968 006
- US-A- 6 022 316
- US-A1- 2003 065 294
- US-A1- 2003 073 949
- US-A1- 2005 118 705
- US-A1- 2007 009 474
- US-A1- 2008 220 092
- US-A1- 2008 220 092
- US-A1- 2009 234 269
- ASH W L ET AL: "Computer simulations of membrane proteins", BIOCHIMICA ET BIOPHYSICA ACTA. BIOMEMBRANES, AMSTERDAM, NL, vol. 1666, no. 1-2, 3 November 2004 (2004-11-03), pages 158-189, XP004617561, ISSN: 0005-2736, DOI: 10.1016/J.BBAMEM.2004.04.012
- MARK R PRAUSNITZ ET AL.: 'Electroporation of mammalian skin: A mechanism to enhance transdermal drug delivery' PROC. NATL. ACAD. SCI. vol. 90, November 1993, pages 10504 - 10508, XP000601520
- AJAY K. BANGA ET AL.: 'Assessing the potential of skin electroporation for the delivery of protein- and gene- based drugs' TIBTECH vol. 16, October 1998, pages 408 - 412, XP004145647

## Description

### FIELD OF THE INVENTION

The present invention relates to system for delivering a treatment agent into human skin.

Embodiments of the invention have been particularly developed for delivering a skin treatment agent below the stratum corneum of human skin. While some embodiments will be described herein with particular reference to that application, it will be appreciated that the invention is not limited to such a field of use, and is applicable in broader contexts. For example, other embodiments are used to deliver agents for treating the tissue underlying the skin.

### BACKGROUND

Any discussion of the background art throughout the specification should in no way be considered as an admission that such art is widely known or forms part of common general knowledge in the field.

Electroporation has been primarily used to temporarily influence the permeability of the membranes of cells to allow for the insertion of additional compounds to those cells. It has also been recognized for its ability to create temporary micro-channels through the layers of the skin that are known as "aquaporins". More recently, work has been undertaken to use electroporation for the percutaneous administration of therapeutic drugs - that is, the delivery of drugs transdermally. The transdermal delivery is preferred because it is relatively non-invasive and safe.

Known methods of elecroporation for transdermal drug delivery are, however, expensive to administer effectively and required skilled personnel to perform the delivery. This becomes particularly problematic for those treatment regimes that required repeated or periodic repeats of the process.

In a prior art method, a liquid or gel containing the treatment agent is manually applied to a surface of the skin and physically spread across the stratum corneum by a human operator. Following from this, a hand held emitter, which emits the electroporation field, is positioned by the operator to engage with and then to be progressed across the surface and the liquid or gel lying on the surface. This method suffers from a number of distinct disadvantages. For example, there is considerable risk of cross-contamination of components and, in particular, a high risk of contamination of the treatment agent.

As a possible solution to this cross-contamination issue is to first induce the electoporation in the skin and then quickly apply the drug or other treatment agent to the surface of the skin. However, this too has proved unsuccessful, as the surface of the skin - and more particularly, the stratum corneum - swiftly returns to its less porous state once the inducement is removed.

Another known approach is to provide a combined emitter/applicator to allow the simultaneous application to a surface of the patient's skin of both the electroporation field and a gel including the treatment agent. While this approach may address the timing problem, there remains considerable risk of cross-contamination. Moreover, the combined emitter/applicator is relatively complex, expensive to manufacture and maintain, and difficult to keep clean.

US Patent No. 5,968,006 discloses an apparatus and method for transdermal molecular delivery, comprising a first electrode assembly having an anode and a cathode in closely spaced relation for engaging the stratum corneum through which to apply an electric field, a second electrode assembly spaced from the first electrode assembly through which to apply an electric field and comprising at least one of an anode and a cathode, a first power supply including a first circuit preferably connected to the first electrode assembly for applying a pulsed electric field of sufficient amplitude to induce pores in the stratum corneum, second power supply including a second circuit connected to the first electrode assembly and the second electrode assembly for applying a low voltage continuous electric field of a preselected polarity and sufficient amplitude to induce migration of molecules through pores in the stratum corneum. The iontophoresis current density to the electrode assembly is around 0.5 mA per cm², and after an electroporation pulsing event, between around 2.9 and 7.0 mA per cm².

US Patent Application Publication No. US 2003/0073949 A1 discloses a non-invasive method of enhancing the permeability of the skin to a biologically active permeant or compound utilizing a combination of sonophoresis and chemical enhancers. Synergism brought simultaneously applying iontophoresis, electroporation, mechanical vibrations and magnetophoresis is used to optimize the transcutaneous active permeation of compounds, considerably lowering the time of treatment. The method is intended also for, among others, the non-invasive painless treatment of cellulitis, localized fat, stretch marks and flacid skin.

US Patent No. 6,022,316 discloses an apparatus and a method for electroporating tissue. At least one micropore is formed to a predetermined depth through a surface of the tissue, and electrical voltage is applied between an electrode electrically coupled to the micropore and another electrode spaced therefrom. By applying electroporation to tissue that has been breached by a micropore, the electroporation effects can be targeted at tissue structures beneath the surface, such as capillaries, to greatly enhance the withdrawal of biological fluid, and the delivery for uptake of compounds into the tissue. In a preferred embodiment, a device is provided having elements that are suitable for microporating the tissue and which serve as the electroporation electrodes.

### SUMMARY OF THE INVENTION

It is an object of the present invention to overcome or ameliorate at least one of the disadvantages of the prior art, or to provide a useful alternative.

According to a first aspect of the invention there is provided an electroporation device (11) for delivering a treatment agent into human skin (3), the device including: a pump (4) for creating a flow (6) of droplets including the treatment agent; a nozzle (5) for directing the flow toward a surface (7) of the skin (3) to be treated; and an electroporation transducer (17) for forming pores in the surface (7) for allowing at least some of the droplets to pass through the surface (7) and into the skin (3) characterized in that the droplets have a diameter less than the diameter of the pores.

It will be appreciated that the formation of aquaporins by electroporation can be equated to the formation of pores or pseudo-pores in the skin. As such, aquaporins can sometimes be referred to as pores. However, aquaporins more accurately function as regulators of the flow of water and are now known to be separate and distinct from pores.

In an embodiment the droplets are minute.

In an embodiment, the droplets have a size that is smaller than the size of a notional droplet of the same material held together by natural surface tension. That is, the natural surface tension is not the mechanism for determining the size of the droplets used in the embodiments of the invention. In this specification, the action of producing the droplets of such as smaller size is referred to as fractionating the natural surface tension of a notional droplet of the same material.

In an embodiment, the skin includes a stratum corneum in which the pores form.

In an embodiment, the skin includes target tissue that lies below the stratum corneum and the pores allow at least some of the droplets to pass through the stratum corneum to the target tissue.

In an embodiment, the skin includes cells that form pores, wherein the pores are hydrophilic pores.

In an embodiment, the flow is directed toward the surface just prior to exposing the surface to the electroporation field.

In an embodiment, the flow is directed toward the surface just after exposing the surface to the electroporation field.

In an embodiment, the flow is directed toward the surface during the exposing of the surface to the electroporation field.

In an embodiment, the flow is directed toward the surface during more than one of the above sequences.

In an embodiment, the flow includes a solute and a solvent. In an embodiment, the solute is the treatment agent and the solvent is water. In another embodiment, the flow includes two or more liquids, at least one of which includes the treatment agent.

In an embodiment, the electoporation field emanates from an emitter that is spaced apart from the surface. In other embodiments, the emitter is abutted with the surface. In further embodiments the emitter is temporally abutted and spaced apart from the surface.

In an embodiment, the flow is directed toward the skin prior to the skin being exposed to the field. In an embodiment, the flow is directed toward the skin while the skin is being exposed to the field. In an embodiment, the skin is exposed to the field prior to the flow being directed toward the skin. In another embodiment the skin is exposed to the field and the flow contemporaneously.

In an embodiment, the pump includes a reservoir for containing the treatment agent. In an embodiment, the reservoir includes a bulk liquid having a solute and a solvent, wherein the solute includes the treatment agent.

In an embodiment, the flow comprises a discrete dose of the treatment agent. In an embodiment, the pump provides successive substantially like doses.

The pump and nozzle ensure that the nominal diameter of the droplets is small, and certainly smaller than would occur if natural surface tension were the mechanism determining that diameter.

In an embodiment, the flow is created during a first interval and the transducer forms the aquaporins in a second interval wherein the second interval follows substantially immediately from the first interval.

In an embodiment, the intervals overlap.

Reference throughout this specification to "one embodiment", "some embodiments" or "an embodiment" means that a particular feature, structure or characteristic described in connection with the embodiment is included in at least one embodiment of the present invention. Thus, appearances of the phrases "in one embodiment", "in some embodiments" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

As used herein, unless otherwise specified the use of the ordinal adjectives "first", "second", "third", etc., to describe a common object, merely indicate that different instances of like objects are being referred to, and are not intended to imply that the objects so described must be in a given sequence, either temporally, spatially, in ranking, or in any other manner.

In the claims below and the description herein, any one of the terms comprising, comprised of or which comprises is an open term that means including at least the elements/features that follow, but not excluding others. Thus, the term comprising, when used in the claims, should not be interpreted as being limitative to the means or elements or steps listed thereafter. For example, the scope of the expression a device comprising A and B should not be limited to devices consisting only of elements A and B. Any one of the terms including or which includes or that includes as used herein is also an open term that also means including at least the elements/features that follow the term, but not excluding others. Thus, including is synonymous with and means comprising.

### BRIEF DESCRIPTION OF THE DRAWINGS

Preferred embodiments of the invention will now be described, by way of example only, with reference to the accompanying drawings in which:
Figure 1 is a schematic representation of an applicator for the treatment agent applied according to one embodiment; and
Figure 2 is a schematic representation of an emitter used in the method of Figure 1.

### DETAILED DESCRIPTION

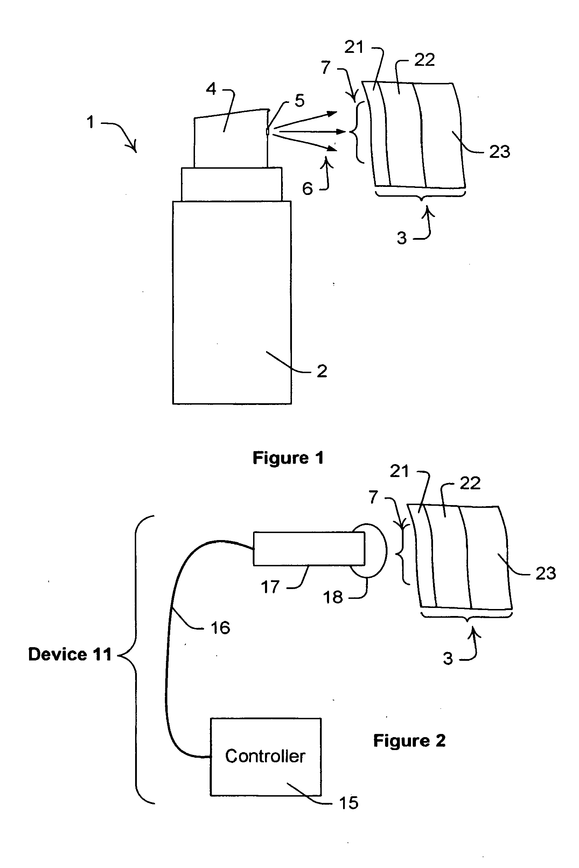

Reference is made to Figure 1 where there is illustrated schematically part of an apparatus for delivering a treatment agent. The apparatus includes a hand actuated pneumatic atomizer 1 that has a 7 ml generally cylindrical reservoir 2 for containing a bulk liquid (not shown) that includes a predetermined concentration of the treatment agent. In this embodiment, the bulk liquid includes a solvent in the form of water and a treatment agent in the form of hyaluric acid. The treatment agent is intended for delivery into human skin 3. Atomizer 1 includes a finger actuated pump 4 for creating a flow of minute droplets including the treatment agent. It will be appreciated that this flow is drawn from the bulk liquid in reservoir 2 in a sequence of doses and is supplied sequentially to a nozzle 5 for directing the flow 6 to a surface 7 of the skin 3.

In other embodiments other treatment agents, or combinations of treatment agents, are used. For example, some treatment agents are selected to provide nutrient feed to the target cells during cell reproduction. In other embodiments use is made of treatment agents that include one or more other active agents.

The volume and form of atomizer 1 is provided by way of example only. In other embodiments alternative volumes and forms are used. In the present embodiment the volume chosen is suitable for the specific liquid being applied, and the form chosen provides for ease of manual application.

It will be appreciated that surface 7 is a portion of the entire surface of skin 3. As will be described below, a treatment of the skin includes in some embodiments the sequential treatment of a plurality of surfaces 7 of skin 3. However, in other embodiments, a treatment involves only a single surface 7.

As best shown in Figure 2, the apparatus of the embodiment also includes an electroporation device 11 for forming hydrophilic aquaporins (not shown) in the skin that allow at least some of the droplets to pass into the skin. It will be appreciated that the aquaporins formed in the stratum corneum by the electroporation field are relatively large and allow the passage of a high proportion of the minute droplets, together with the included treatment agent, into and through the stratum corneum and to the underlying skin layers or other target tissue.

Atomizer 1 does not make use of a propellant to minimize the need to use foreign agents or volatile materials. Rather, atomizer 1 is manually actuated while disposed near skin to provide a small flow only - typically about 0.25 ml - to surface 7. The application of mechanical force to generate the flow, and the absence of a propellant, reduces the risk of any chemical change to the treatment agent while also providing the minute drops on the surface having a smaller nominal diameter than would result from natural surface tension mechanisms.

The droplets in the flow remain substantially separated and individually wet the surface 7. A single actuation of the above volume is typically sufficient to provide a distribution of fine droplets across surface 7. If required, additional doses are able to be applied by the operator through successive actuations of pump 4.

The inventor has found that as additional doses are added and the combined volume of liquid deposited on surface 7 increases, the individual droplets start to pool together, and are held together by natural "un-fractionated" surface tension. Each larger pool is less able to pass through the aquaporins created by the electroporation field, and results in lower rates of passage of the treatment agent into skin 3. That is, the formation of larger droplets and pools leads to the associated treatment agent being effectively trapped above surface 7. Accordingly, the method of the preferred embodiments encourages the operator to use time separated doses of minute droplets, which best ensures the droplets will not pool and hence, maintain a nominal diameter that is the same or smaller than the aquaporins created through electroporation. This has the advantage of also ensuring that only relatively small doses of the treatment agent are required as the applied volume of fluid is also small. This, in turn, allows costs to be better contained - for the treatment agent is often expensive - and for an improvement in the efficacy of the treatment being provided.

The minute droplets - which are small relative to the aquaporins being created - are but fractions of prior art drops, creams, or other applications. This fractionation of those prior art droplets allows the surface tension problem of the prior art to be avoided.

The prior art attempts to deliver liquids and gels to the deeper layers of the skin are substantively compromised by the size of the smallest drops of those liquids and gels being determined, in effect, by their different but naturally occurring surface tensions. The result being that, with diameter of the aquaporins being considerably smaller, there is little or no delivery of the desired material.

In the context of this specification it will be appreciated that atomizer 1 coverts the bulk liquid into the flow of minute separate droplets. This flow is a spray or mist, where the droplet size has a distribution typically spanning from micron size to finer. However, the droplets are not atomic sizes, notwithstanding the term "atomizer". Another term that is able to be used to describe the action of atomizer 1 is the nebulization of the bulk fluid or a surface tension fractionater.

With the methodology of the preferred embodiments only a small amount of the treatment agent is required due to the high rate of passage of the agent into the skin. Accordingly, reservoir 2 of atomizer 1 need only be small in volume. While use is made in the specifically described embodiment of reservoir 2 containing 7 ml of bulk fluid, in other embodiments different volumes are contained, and different reservoir sizes are used. Other frequently used reservoir sizes for the treatment of facial skin surfaces include a capacity of 5 ml and 9 ml respectively. For treating surfaces having a larger surface area - for example, thighs and buttocks - it is more usual to provide a reservoir having a capacity of 20 ml.

Atomizer 1 is constructed predominantly of inert plastics and is supplied to the operator in a sealed wrapper and with a tamper-evident cap or other closure. The wrapper and closure are removed by the operator just prior to use. Accordingly, the risk of chemical change of the treatment agent is small. Moreover, as the treatment agent is only exposed to air for a short duration before being passed into the skin, this reduces the risk of impurities being introduced not only into the treatment agent, but also into the skin.

Device 11 includes a controller 15 that is mains power supplied and which generates an electroporation signal which is provided by electrical lead 16 to a hand held transducer or emitter in the form of a wand 17. The wand is responsive to the signal for generating an electroporation field that emanates from a radiating element 18 at one end of wand 17. The field is comprised of an electromagnetic pulse in the RF range. However, the amplitude is variable, as too is the duty cycle and period of the pulse to allow for different treatment agents, different skin types, and desired depths of penetration of the skin. For example, for a treatment that is to be passed into the skin of the face, the desired depth of penetration of the treatment agent will be less than, say, where the treatment is of cellulite on the thigh. Additionally, regard is had to the skin type and age, which are also factors in skin depth.

Surface 7 is an outer surface of the skin - that is, the outer surface of the stratum corneum 21 - and has an area of about 30 to 50 mm in diameter. In other embodiments, surface 7 has a different diameter or is other than generally circular. This surface 7 is cleaned prior to performing the method of the invention. The cleaning is simply to remove any foreign matter for the surface of the skin. In this embodiment, use is made of a specific pre-treatment of exfoliating some of the stratum corneum 21 with an enzyme exfoliator. In another embodiment, use is made of a cleaner that also provides an antibacterial agent.

The strateum corneum 21 lies above the dermis 22, while the latter lies above the hypodermis 23. Below the hypodermis 23 lies bone and/or muscle. For the embodiments of the invention that are directed to skin treatments, as opposed to therapeutic drug treatments, the desired penetration of the treatment agent is at least through the stratum corneum and to the dermis. For some treatments, the desired penetration is deeper.

It is understood that the skin includes many more layers than those explicitly illustrated in the Figures. The additional layers have been omitted for the sake of clarity of illustration.

In use, an operator identifies the skin that is to be treated. Typically, the skin concerned will define a plurality of contiguous surfaces 7 that are to be treated in succession. However, for some persons, the surfaces 7 will be other than contiguous. For example, in some instances a person has spaced apart scarring or wrinkles that are to be treated.

Once a first surface 7 is identified, the operator positions atomizer 1 such that nozzle 5 is opposed to and spaced apart by about 40 mm from surface 7. A single finger actuation of pump 4 is provided by the operator to force the flow through nozzle 5 and toward surface 7. The minute droplets in the flow are thereby distributed across surface 7. As mentioned above, only a light application of the flow is required. As a guide, surface 7 should have a sheen that is visibly observable due to the presence of the droplets. If surface 7 is not sufficiently wetted to provide a visible sheen, the operator applies a further dose. As each dose provided by atomizer 7 is small, this allows the operator to progressively apply the doses until the wetness or sheen is observed. Once there is a distribution of the minute droplets across surface 7 the operator progresses to the next step. However, if the operator notices any movement of liquid on surface 7, too many droplets have been applied and have started to pool. In this latter circumstance, at least some of the liquid is then removed - for example, by dabbing with an absorbent cloth - to improve the proportion of the treatment agent that is subsequently readily available for passage into skin 3.

With the requisite distribution of minute droplets applied to surface 7, the operator then places wand 17 such that element 18 is directly opposed to but about 20 to 30 mm spaced apart from the surface. This configuration is illustrated schematically in Figure 2. In other embodiments wand 17 is maintained closer to the surface. In still further embodiments, wand 17 is lightly abutted against and moved along the surface. In even further embodiments, a combination of the above configurations is used during a given application.

When controller 15 is actuated, an electroporation field subsequently emanates from element 18. This field, in this embodiment, is a pulsed field, and is applied for anywhere between about 10 seconds to continuously throughout the treatment to allow for the passage of the droplets into skin 3. In determining the duration of the field, the operator has regard to the treatment agent, the solvent or other liquid used to carry the treatment agent, the nature of the treatment, the sheen of the skin provided by the remaining droplets, and the elapsed time.

Following the treatment of surface 7, the operator defines the next surface 7 and follows the same methodology to complete that treatment. This is repeated until all of the defined surfaces 7 are treated.

For some treatments, use is made of a plurality of treatment agents that are applied in succession to surface 7 alternately with the application of the electroporation field. This allows the passage into skin 3 of different treatment agents. In some cases this allows, during a single treatment session, the application of two treatment agents that would have otherwise have had to be applied during separate treatment sessions. This occurs with the prior art, for example, where the two different treatment agents react with each other and, hence, cannot be exposed to each other.

In this embodiment the solvent and solute is a suspension of many different components that are buffered by a buffering agent. Preferably, the buffering agent is an oil or oil-based material that isolates the different solutes from each other while stored in the reservoir.

It will be appreciated that the flow is created during a first interval and wand 17 forms the aquaporins in a second interval wherein the second interval follows substantially immediately from the first interval. However, in some embodiments the intervals overlap, even if only slightly. In other embodiments, the second interval falls between two first intervals.

The major advantages of the embodiments of the invention include:
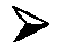 Minute droplets are created of the required size, in a timely manner, for application and transmission through the aquaporins.
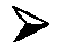 Only very small volumes of the flow treatment agent are required.
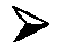 Wastage of the often expensive treatment agent is low due to the high rates of passage of the treatment agent into the skin. (This, in turn, being assisted by the use of small doses that are applied to a specific surface).
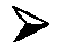 Ease of repeatability of the methodology between skin surfaces, persons, and times.
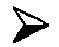 Low risk of cross-contamination of the treatment agent due to there being:
   - No physical contact with the skin by the applicator of the flow. That is, neither atomizer 1 nor nozzle 5 need directly physical contact surface 7.
   - No physical contact with the skin by the operator.
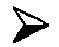 For those embodiments where there is a need for more complete physical separation, it is possible to perform the application with no physical contact with the skin by the electroporation device. That is, element 18 of wand 17 need not directly physical contact surface 7.
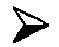 Ease of training of operators.
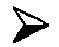 The ability to progressively increase the volume of the treatment agent on the skin to be within the required range, and the ability to assess this visually.
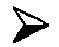 Small quantities of the treatment agent only need to be provided due to high transmission rates, low wastage, and ease of use by an operator. This allows the treatment agent to be provided in an applicator that is sealed and single use, which in turn reduces the risk of cross-contamination.
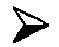 No propellant is used, which reduces the risk of contamination of the treatment agent by such a propellant.

Use of the above described methodology is particularly suitable for treatment agents that are water soluble or which are water-based. It is also suitable for treatment agents containing oils or lipids.

It is possible to achieve high transmission rates of the treatment agent into and through the stratum corneum due to combining electroporation techniques with the direct application of small quantities of minute droplets onto the skin surface. The droplets wet the surface, but are not applied in sufficient quantity to allow substantive pooling of the droplets. That is, the surface is wetted, not saturated.

In addition, the capital costs are relatively modest compared with prior art systems.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the above description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the Detailed Description are hereby expressly incorporated into this Detailed Description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those skilled in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order riot to obscure an understanding of this description.

## Claims

1. An electroporation device (11) for delivering a treatment agent into human skin (3), the device including:
a pump (4) for creating a flow (6) of droplets including the treatment agent;
a nozzle (5) for directing the flow toward a surface (7) of the skin (3) to be treated; and
an electroporation transducer (17) for forming pores in the surface (7) for allowing at least some of the droplets to pass through the surface (7) and into the skin (3)
**characterized in that** the droplets have a diameter less than the diameter of the pores.

2. A device according to claim 1 wherein the surface (7) includes a stratum corneum (21) in which the pores form and target tissue that lies below the stratum corneum (21), wherein the pores allow at least some of the droplets to pass through the stratum corneum (21) to the target tissue.

3. A device according to claim 1 or claim 2 wherein the surface (7) includes cells having pores, wherein the pores are hydrophilic pores.

4. A device according to any one of the preceding claims wherein the flow is directed toward the surface (7) prior to exposing the surface to the electroporation field.

5. A device according to any one of claims 1 to 3 wherein the flow is directed toward the surface (7) while the surface (7) is being exposed to the field.

6. A device according to any one of claims 1 to 3 wherein the surface (7) is exposed to the field prior to the flow being directed toward the surface (7).

7. A device according to any one of the preceding claims wherein the flow includes a solute and a solvent.

8. A device according to claim 7 wherein the solute is the treatment agent and the solvent is water.

9. A device according to any one of claims 1 to 7 wherein the flow includes two or more liquids, at least one of which includes the treatment agent.

10. A device according to any one of the preceding claims wherein the electoporation field emanates from an emitter (18) that is spaced apart from the surface (7).

11. A device according to any one of the preceding claims wherein the pump (4) includes a reservoir (2) for containing the treatment agent.

12. A device according to claim 11 wherein the reservoir (2) includes a bulk liquid having a solute and a solvent, wherein the solute includes the treatment agent.

13. A device according to any one of the preceding claims wherein the flow is created during a first interval and the transducer (17) forms the pores in a second interval wherein the second interval follows substantially immediately from the first interval.

## Patentansprüche

1. Elektroporationsvorrichtung (11) zur Abgabe eines Behandlungsmittels in die menschliche Haut (3), wobei die Vorrichtung Folgendes umfasst:
eine Pumpe (4) zur Erzeugung eines Stromes (6) von Tröpfchen, die das Behandlungsmittel umfassen;
eine Düse (5) zum Richten des Stromes auf eine Oberfläche (7) der Haut (3), die behandelt werden soll;
und
einen Elektroporationswandler (17) zur Bildung von Poren in der Oberfläche (7), damit wenigstens einige der Tröpfchen durch die Oberfläche (7) und in die Haut (3) passieren können,
**dadurch gekennzeichnet, dass** die Tröpfchen einen Durchmesser aufweisen, der geringer als der Durchmesser der Poren ist.

2. Vorrichtung nach Anspruch 1, wobei die Oberfläche (7) ein Stratum corneum (21) umfasst, in welchem sich die Poren bilden, und Zielgewebe, das unter dem Stratum corneum (21) liegt, wobei die Poren wenigstens einige der Tröpfchen durch das Stratum corneum (21) zum Zielgewebe passieren lassen.

3. Vorrichtung nach Anspruch 1 oder Anspruch 2, wobei die Oberfläche (7) Zellen umfasst, die Poren aufweisen, wobei die Poren hydrophile Poren sind.

4. Vorrichtung nach einem der vorangehenden Ansprüche, wobei der Strom auf die Oberfläche (7) gelenkt ist, bevor die Oberfläche dem Elektroporationsfeld ausgesetzt wird.

5. Vorrichtung nach einem der Ansprüche 1 bis 3, wobei der Strom auf die Oberfläche (7) gerichtet wird, während die Oberfläche (7) dem Feld ausgesetzt wird.

6. Vorrichtung nach einem der Ansprüche 1 bis 3, wobei die Oberfläche (7) dem Feld ausgesetzt wird, bevor der Strom auf die Oberfläche (7) gerichtet wird.

7. Vorrichtung nach einem der vorangehenden Ansprüche, wobei der Strom einen gelösten Stoff und ein Lösungsmittel umfasst.

8. Vorrichtung nach Anspruch 7, wobei der gelöste Stoff das Behandlungsmittel ist und das Lösungsmittel Wasser ist.

9. Vorrichtung nach einem der Ansprüche 1 bis 7, wobei der Strom zwei oder mehrere Flüssigkeiten umfasst, wobei wenigstens eine davon das Behandlungsmittel umfasst.

10. Vorrichtung nach einem der vorangehenden Ansprüche, wobei das Elektroporationsfeld von einem Emitter (18) abgestrahlt wird, der in einem Abstand von der Oberfläche (7) angeordnet ist.

11. Vorrichtung nach einem der vorangehenden Ansprüche, wobei die Pumpe (4) einen Speicher (2) umfasst, der das Behandlungsmittel enthält.

12. Vorrichtung nach Anspruch 11, wobei der Speicher (2) einen Flüssigkeitskörper umfasst, der einen gelösten Stoff und ein Lösungsmittel aufweist, wobei der gelöste Stoff das Behandlungsmittel umfasst.

13. Vorrichtung nach einem der vorangehenden Ansprüche, wobei der Strom während einem ersten Intervall erzeugt wird und der Wandler (17) die Poren in einem zweiten Intervall bildet, wobei das zweite Intervall im Wesentlichen unmittelbar nach dem ersten Intervall folgt.

## Revendications

1. Dispositif d'électroporation (11) destiné à l'administration d'un agent de traitement dans la peau (3) d'un humain, le dispositif comprenant :
une pompe (4) destinée à créer un écoulement (6) de gouttelettes comprenant l'agent de traitement ;
une buse (5) destinée à diriger l'écoulement vers une surface (7) de la peau (3) à traiter ; et
une tête émettrice par électroporation (1 7) destinée à former des pores dans la surface (7) pour permettre au moins à certaines gouttelettes de traverser la surface (7) pour aller jusque dans la peau (3),
**caractérisé en ce que** les gouttelettes ont un diamètre inférieur au diamètre des pores.

2. Dispositif selon la revendication 1, dans lequel la surface (7) comprend un stratum corneum (21) dans lequel les pores se forment et un tissu cible qui se trouve en dessous du stratum corneum (21), les pores permettant au moins à certaines gouttelettes de traverser le stratum corneum (21) pour aller jusqu'au tissu cible.

3. Dispositif selon la revendication 1 ou la revendication 2, dans lequel la surface (7) comprend des cellules qui présente des pores, les pores étant des pores hydrophiles.

4. Dispositif selon l'une quelconque des revendications précédentes, dans lequel l'écoulement est dirigé vers la surface (7) avant d'exposer la surface au champ d'électroporation.

5. Dispositif selon l'une quelconque des revendications 1 à 3, dans lequel l'écoulement est dirigé vers la surface (7) tandis que la surface (7) est exposée au champ.

6. Dispositif selon l'une quelconque des revendications 1 à 3, dans lequel la surface (7) est exposée au champ avant que l'écoulement ne soit dirigé vers la surface (7).

7. Dispositif selon l'une quelconque des revendications précédentes, dans lequel l'écoulement comprend un soluté et un solvant.

8. Dispositif selon la revendication 7, dans lequel le soluté est l'agent de traitement et le solvant est de l'eau.

9. Dispositif selon l'une quelconque des revendications 1 à 7, dans lequel l'écoulement comprend deux liquides ou plus, dont au moins un comprend l'agent de traitement.

10. Dispositif selon l'une quelconque des revendications précédentes, dans lequel le champ d'électroporation émane d'un émetteur (18) qui est situé à distance de la surface (7).

11. Dispositif selon l'une quelconque des revendications précédentes, dans lequel la pompe (4) comprend un réservoir (2) destiné à contenir l'agent de traitement.

12. Dispositif selon la revendication 11, dans lequel le réservoir (2) comprend un liquide en vrac ayant un soluté et un solvant, le soluté comprenant l'agent de traitement.

13. Dispositif selon l'une quelconque des revendications précédentes, dans lequel l'écoulement est créé durant un premier intervalle et la tête émettrice (17) forme les pores dans un second intervalle, le second intervalle suivant sensiblement immédiatement le premier intervalle.
